# EUROPEAN PATENT SPECIFICATION

(11) **EP 4 284 571 B1**
(45) Date of publication and mention of the grant of the patent: **05.03.2025**
(21) Application number: 22705133.1
(22) Date of filing: 26.01.2022
(51) Int. Cl.: B09B 3/00, B09B 5/00, A01K 67/033, C05F 17/00

(54) **INTELLIGENT DEVICE FOR COMPOSTING ORGANIC WASTE BY USING LARVAE OF SAPROPHAGOUS INSECTS AND PROCESS FOR MONITORING BIOCONVERSION**
INTELLIGENTES GERÄT ZUR KOMPOSTIERUNG ORGANISCHER ABFÄLLE DURCH DIE VERWENDUNG VON SAPROPHAGUS-LARVEN UND EINEM BIOKONVERSIONSÜBERWACHUNGSVERFAHREN
DISPOSITIF INTELLIGENT DE COMPOSTAGE DE DECHETS ORGANIQUES PAR L'UTILISATION DE LARVES DE SAPROPHAGES ET PROCEDURE DE SUIVI DE LA BIOCONVERSION

(30) Priority: 27.01.2021 IT 202100001571
(43) Date of publication of application: 06.12.2023
(73) Proprietor: Circle Biotech Srl, 70124 Bari (BA) (IT)
(72) Inventor: CAPRIO, Francesco, 70010 Valenzano (BA) (IT)
(74) Representative: De Tullio, Michele Elio
(86) International application number: PCT/IB2022/050659
(87) International publication number: WO 2022/162538

(56) References cited:
- WO-A2-2012/029041
- CN-A- 109 174 919
- ES-A1- 2 331 452
- KR-A- 20160 120 540
- US-A1- 2003 143 728

## Description

The present invention relates to the field of organic waste treatment apparatuses and devices; in particular, the present invention relates to a device for biologically converting domestic and community's organic waste based on the use of larvae of saprophagous insects, in particular dipterans.

In particular, the present invention discloses an intelligent composter provided with a control unit and a system of sensors configured for assessing the operation of the composter and the quality of the bioconversion by counting the migrating larvae, the assessment of the larval stage of the migrating larvae and the temperature and humidity conditions present inside the device.

### Present status of the art

Numerous devices and numerous patent documents are present in the status of the art, describing composting devices that use larvae of dipterans and comprise the breeding of said larvae.

Many of the devices known in the present status of the art are provided with sensors aiming at assessing the environmental conditions present inside the composter.

Document FR3013561 entitled "*Dispositif pour l'elevage d'insectes volants tels que hermetia illucens*" relates to a device for breeding flying insects of Hermetia Illucens or a similar type, comprising at least one first so-called chamber (1) comprising at least one shelf (2) and receive trays (3) where there are placed pupae of insects, a heater blower (4), and a humidifier (5), at least one second so-called egg laying chamber (6) comprising at least one sieve (7) horizontally partitioning said egg laying chamber (6) into an upper portion (6a) called aviary and a lower portion (6b), called collector, said sieve (7) featuring a number of holes whose dimensions are just greater than the dimensions of the eggs and larvae of insects, whose dimensions are smaller than the dimensions of the insect imagoes, and a pipe (8) which connects the pupae chamber (1) to the upper portion (6a) of the egg laying chamber (6); this device relates to the field of industrial or domestic bioconversion and in particular the field of breeding of Hermetia Illucens, and allows to degrade organic waste, in particular into a compost. This device possibly comprises sensors, for example sensors used to monitor temperature and humidity, conditioning means, and control means and electromechanical actuators.

Document KR101010316 entitled "Apparatus for treatment of organic waste using BSF larva" discloses an organic waste treatment container which uses a larva of black soldier fly, including a main body (120), a collection portion (130), and a container cover. The upper surface of the main body of the container is open, and one or several tilted surfaces are formed on one side of the main body. The collection portion is formed on an outer side of the tilted surface of the main body of the container. The cover of the container opens and closes the upper portion of the main body of the container. An organic waste treatment device includes a main body of the structure, a part of the leg of the main body, and a collector; this document discloses a reproduction container and an apparatus for breeding domestic and non-domestic larvae, which can decompose the organic waste produced by families, blocks of flats, canteens, etc. in an eco-friendly manner. This invention claims automated reproduction processes (for example, collection), the use of sensors for measuring the environmental conditions, and process control and climatic conditioning means.

Document JP2010110307 entitled "Insect-raising compost bed" discloses an insect reproduction device, characterized in that it comprises a treatment container of a stacking growth type, complete with humidity and temperature conditioning. This invention is characterized by the presence of measurement and assessment means and communication means to transfer information to a personal computer. This invention provides data filing facilities and makes it possible to display an analysis of the operating conditions and is provided with a planning control software for controlling the reproduction status.

Document WO2019053439 entitled "Insect Larvae Breeding" relates to breeding of insect larvae. In particular, it is an insect-larvae breeding system that can be assembled by using one or several modules for preparing food for insect larva. This system includes the use of an automated environmental conditioning and a specifically developed system of sensors.

Patent application CN109174919 entitled "Device and process for a continuous and integral treatment of perishable waste by using black flies" discloses a device and a process for a continuous and integral treatment of perishable waste through the use of black flies and relates to the technical field of perishable waste treatment by means of insects. In a continuous and integrated treatment device, perishable waste is continually added every day, the black soldier fly larvae are added by lots. As the black soldier fly larvae belonging to different lots grow and reach a prepupal period, thanks to their characteristic mobility, the black soldier fly larvae leave the reactor through a structure featuring a tilted face in order for them to be collected.

Patent application WO2012029041 entitled "Plant and method for bioconversion of organic waste and biostabilization of municipal waste" discloses a system for biologically treating organic waste by using insect larvae, comprising a tank provided with a removable cover and ventilation and temperature control means suitable for inducing larvae to migrate to outside the waste mass whenever they are in a desired larval development stage, means for distributing the individual layers of waste several times and for removing them once the treatment is finished, and means for distributing larvae inside the tank and for collecting and storing larvae at the end of the treatment. The method used for biologically treating organic waste by way of larval stage insects includes a step whereby a first layer of waste is put inside a tank (1), insect larvae or eggs are laid onto said layer, and other layers of waste are added at predetermined time instants or at shorter time intervals with respect to the development time of larvae, thus inducing a spontaneous migration of larvae toward suitable collection areas, under temperature and humidity controlled conditions, and treated waste is removed from the tank.

Patent application ES2331452 entitled "Apparatus and process for disposing organic waste by way of large insects" discloses an apparatus and a process for organic waste disposal by way of insect larvae. This apparatus, specifically designed for using hermetia illucens in treating waste like swine wastewater, comprises a digester (1) wherein a digestion chamber (2) is defined, delimited below by a separation net (3), on which a layer of dry compost (22) is formed; the larvae reach the lower area of the digester during the terminal development stage and are collected in a lower chamber (14) where they are converted into pupae and lay eggs, which are re-integrated in the digester, namely in an egg cap (8).

Patent application US2003143728 (A1) entitled "Disposal apparatus and method for an effective bioconversion of putrescent waste" discloses a method and a system for an efficient bioconversion of putrescent waste into a more usable form. The present invention comprises a generally round shaped container with two small ramps inside the container. In operation, putrescent waste is deposited into the container of the domestic unit. Whenever the container becomes full of larval residues, larvae are removed from the container, the container is emptied of the residues, and larvae are put in the container.

Patent application KR20160120540 (A) entitled "Soldier fly breeding equipment" relates to a soldier fly which enables a device to transform organic waste, such as food waste, into a compost, while larva incubation and mating, egg lying, and imago collection are continually repeated. According to the present invention, larvae fed with food waste in a growth chamber become aged larvae by eating food waste, become chrysalides in a chrysalid chamber, become imagoes, couple together in a coupling chamber, and lay eggs in an egg laying hole formed on a tilted surface. The imago eggs drop down into a lifting chamber and the imago eggs are incubated. Then the process is repeated. Consequently, soldier flies can be continuously reared without feeding additional larvae.

A need comes out from the present status of the art for providing an intelligent device that is capable of monitoring the operation of a composter, by counting the number of larvae present internally thereto and assessing their viability status.

The present invention solves the technical problem of verifying the density of the population of dipteran larvae inside a composter and monitoring the quality of bioconversion by looking at several parameters, namely:
- number of migrating larvae;
- larva larval stage, dimensions, and colour;
- environmental parameters inside the composter.

### Summary of the invention

An object of the present invention is to provide an intelligent device for composting an organic material by way of larvae of saprophagous insects, in particular dipterans, capable of monitoring the density of said larvae through the use of a control unit and a number of sensors, hence the effectiveness of the composting process itself, by assessing the number of migrating larvae and the actual larval status, and the environmental conditions existing inside the device.

In particular, the device according to this invention mainly solves the technical problem of monitoring the number of migrating larvae; as a matter of fact, it is known that, in a composter, the effectiveness of bioconversion depends on the status of the population of dipterans that are fed with the organic substance to be bioconverted; the status of this population depends on season and on climatic conditions.

The device according to this invention is configured in such a way that also dipterans found in nature can lay eggs in the cover, where from larvae can be hatched used to feed the population of dipterans present in the composter.

In those months in which said dipterans are active in nature, larvae, once eggs are hatched, can fall inside the composter and perform their bioconversion agent functions. Vice versa, in those months in which the wild populations are not active, i.e. in the coldest months, dipterans found in nature do not feed the population inside the device, which might affect the operation of the device and the effectiveness of bioconversion negatively; therefore, the present device makes it possible to monitor the bioconversion process and to identify the instants in time when it will be necessary to insert a new lot of larvae from the external world in order to cater for bioconversion. The device according to this invention is a composter characterized in that it is capable of automatically conditioning the environmental conditions internally thereto via ventilation, heating, and heat recovery mechanisms, as well as monitoring the dynamics of the insect population used internally thereto through the use of appropriate sensors and is constantly connected to an operation centre, which monitors the operating status and the quality of bioconversion and delivers new lots of dipterans in the larval stage "on demand", in order to cater for its operation.

The base of the composter is provided with technical compartments in communication with said migration space, capable of receiving dipteran larvae that, having terminated their own growth, migrate in order to find a suitable site where to pupate and perform their metamorphosis; these compartments are also provided with sensors located on their top, properly developed to count the number of larvae dropping toward the compartments themselves, which will be referred to below as "larvameter". Said compartments can also be removed to transfer the larvae accumulated therein.

Said sensors are in communication with a data acquisition and communication device in communication with the external world, which represents the intelligent part of the device according to the present application patent, which will be referred to below as intelligent control unit. Said device, equipped with a software that allows to count those larvae which terminated their own growth cycle inside the composter and "self-collected" together in the technical compartments, present on the bottom thereof, will send a request for new larvae to the operating centre which the composter is in communication with, in order for the staff in charge of maintaining the composters to deliver a new batch of newborn larvae and, if necessary, to collect the larvae at the end of their growth, in order to replace them and make it possible to continue the bioconversion process internally to the composter itself.

### Brief description of the figures

Other advantages, characteristics, and modes of use of the device according to the present invention will be apparent from the following detailed description of one embodiment, which is presented for explanatory, non-limitative purposes only, and with special reference to the attached figures, wherein:

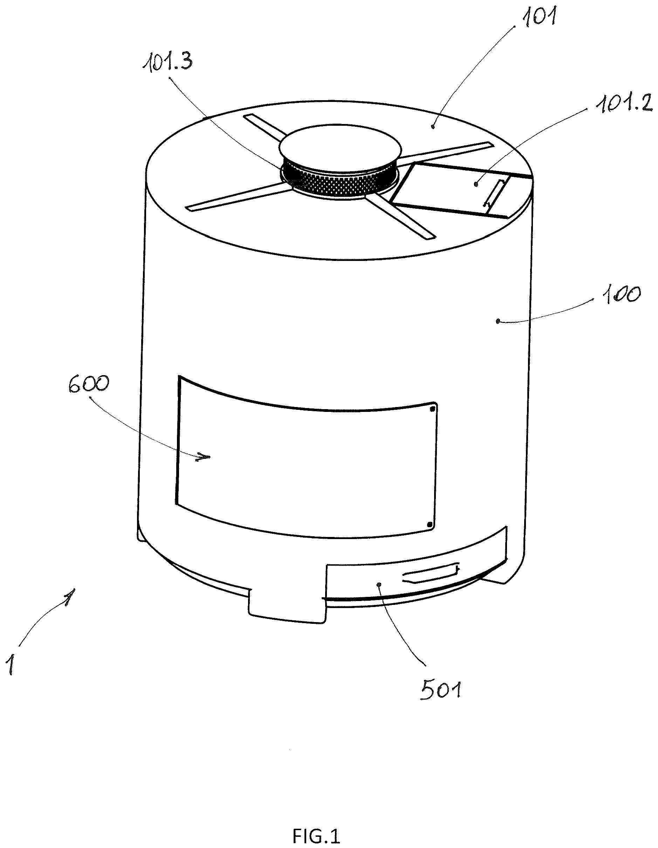

Figure 1 - a front-side axonometric view of an intelligent device for composting organic waste through the use of larvae of saprophagous insects according to one preferred embodiment, the openable portion being in the closed condition;

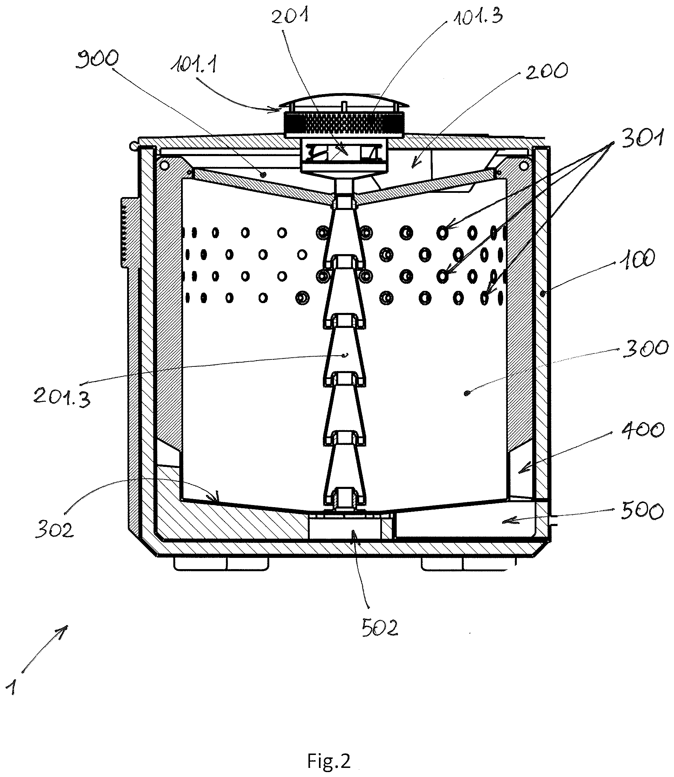

figure 2 - an axial cross-sectional view of the intelligent device for composting organic waste through the use of larvae of saprophagous insects according to one preferred embodiment, wherein the inner portions are visible;

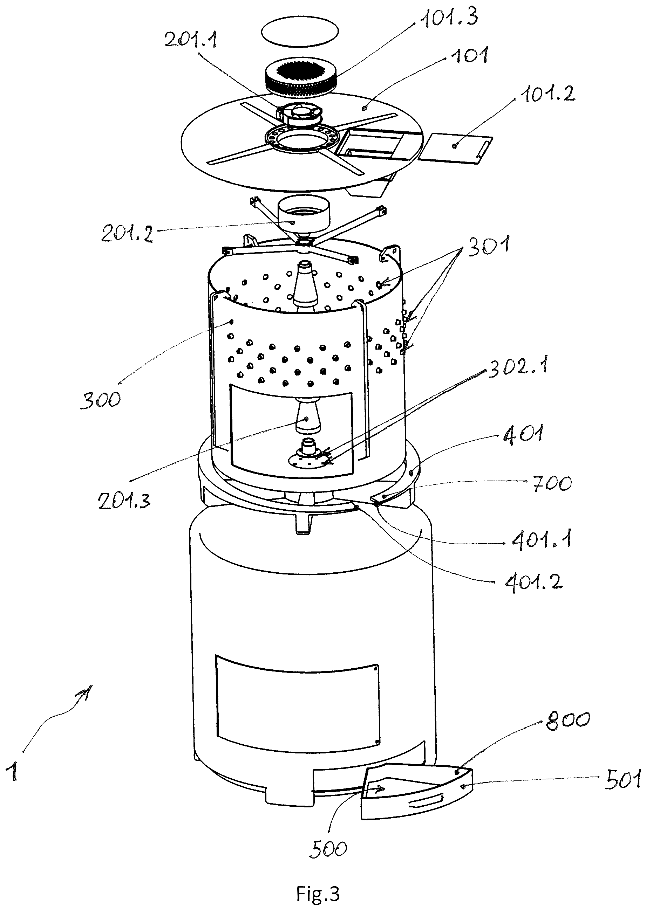

figure 3 - a front side axonometric view of the intelligent device for composting organic waste through the use of larvae of saprophagous insects according to one preferred embodiment in a pre-assembling step.

### Legenda

1. Intelligent device according to the invention;
   100. Outer casing;
      101. cover of the outer casing;
      101.1. air release opening
      101.2. organic waste insertion opening;
      101.3. opening provided with a plurality of holes for laying saprophagous insect eggs;
   200. Inner compartment;
      201. heat exchanger;
      201.1. heat exchanger suction fan;
      201.2. heat exchanger resistor;
      201.3. heat exchange pipe;
   300. Inner container;
      301. Through holes for passage of dipteral larvae;
      302. Lower surface of the inner container;
      302.1 holes on surface 302 for percolate passage;
   400. Migration pipe;
      401. annular ramp element;
      401.1 and 401.2. free ends of the tilted annular element
   500. Lower compartment;
      501. collection drawer;
      502. percolate collection container.
   600. Control unit;
   700. optoelectronic sensor means;
   800. camera means;
   900. temperature and humidity sensor means.

### Detailed description of the invention

The present invention discloses an intelligent device 1 for composting organic waste through the use of larvae of saprophagous insects, comprising an outer casing 100 which delimits an inner compartment 200 suitable for accommodating an inner container 300 coaxially mounted inside the outer casing 100 and suitable for containing organic waste and larvae of saprophagous insects, characterized in that said inner container 300 is open above and is provided with a plurality of through holes 301 in its upper portion, for making it possible for migrating larvae to pass from the inner container 300 to the migration pipe 400 up to reaching a lower collection compartment 500 placed between the outer casing 100 and the bottom 302 of the inner container 300, suitable for collecting the percolate, and in that said device 1 is provided with optoelectronic sensor means 700 capable of detecting the passage of at least one migrating larva from the inner compartment 200 and passing through the migration pipe 400 up to reaching the lower collection compartment 500, and a control unit 600 comprising software means and a wireless module.

The most preferred embodiment of this invention comprises a container featuring a substantially cylindrical shape; this container comprises an outer casing 100, which forms a cover 101, at need removable, in its upper portion.

The device according to this invention is provided with air inlet/outlet means, wild dipteran egg laying means, and air conditioning means connectable to at least one pipe system accommodated in the inner container 300 to cater for air exchange inside to the container.

Said device 1 is structured in such a way as to present an organic waste insertion opening 102 on the cover 101, through which the contents of organic waste bins can be spilled into said composter, thus preventing the inner part of the composter from being exposed.

The cover 101 also comprises two further openings to the external world; the former, placed on the cover top, is used to allow air to come in, which is forced via a suction fan 201.1 placed below the cover 101 to subsequently pass through a heat exchanger 201 placed in the inner compartment 200 below the cover itself; the second opening 103, provided with a plurality of holes arranged in series all throughout the surface of the opening itself and placed under the former one, performs a dual function, i.e. to allow the injected air to go out and simultaneously to make it possible for the populations of wild dipterans, attracted by biotic factors intrinsic to the bioconversion process taking place internally to the composter, to lay their own eggs in said holes.

Once said eggs are hatched, the larvae fall inside the composter in the inner container, thus performing their bioconversion agent function; this process will take place in those months in which said dipterans are active in nature, i.e. in the hot humid months; conversely, in the period when the populations of wild dipterans are inactive, it will be possible to inject newborn larvae through the opening 102 of the cover whenever this is required.

An insulation layer is interposed between the outer casing 100 and the inner container 300 where the injected waste bioconversion process takes place in order to limit the heat exchange with the external environment as much as possible.

The inner compartment delimited by the outer casing 100 and by the cover 102 contains a number of technical measures under the cover 102, which allow to recover the heat possibly produced inside the composter itself.

In particular, a heat exchanger 201 is placed under the cover internally to the inner compartment 200; a heat exchanger sucking fan 201.1, a heat exchanger resistor 201.2, and finally a heat exchanger pipe 201.3 are connected to said heat exchanger 201; said pipe runs in the middle of the inner container 300 and comprises a plurality of superimposed conical elements, capable for distributing the pre-heated air coming from the heat exchanger directly into the waste matrix where said dipteran larvae operate, which consequently will take advantage of a climate suitable for their development and an appropriate oxygenation. Further devices inside the composter include a resistor mounted inside the heat exchanger, capable of further heating the air input to the composter, wherever necessary, and temperature sensors in communication with the intelligent control unit, another task of which is to also control the climatic conditions existing inside the composter.

The inner container 300 where bioconversion takes place is also present internally to said insulation layer.

Bioconversion takes place thanks to the activity of dipteran larvae which feed on the organic waste to grow and digest and convert it into a biomass. Upon terminating their own growth process and being ready to pass to a subsequent larval stage, the larvae feel a need for moving away from the site where they grew and migrating; this migration is due to the need the larvae feel for finding a suitable site where to pupate and perform their metamorphosis.

Said inner container 300 comprises a plurality of through holes 301, in its upper third; said holes are in a direct contact with the waste matrix present in the inner container 300.

These holes 301 make it possible for those larvae which reached this stage and completed their growth process to move away and migrate through a migration pipe 400, which is a gap between the outer casing and the inner container 300.

After entering the migration pipe 400, the larvae will fall down into the collection compartment 500.

In the preferred embodiment of this invention, the migration pipe 400 comprises, internally thereto, an annular element 401 tilted with respect to a horizontal plane, so that it features an opening in the point closest to the vertex, so as to allow for the migrating larvae to slide and pass into a collection drawer 501, and said optical sensors 700 are placed close to the ends 401.1 and 401.2 of said tilted annular element 401.

Said optoelectronic sensor means 700 are placed close to at least one migration route from the inner container 300 to the lower collection compartment 500; these sensors are called "larvameter" sensors.

In the preferred embodiment of the invention, the migration route followed by the larvae from the inner container 300 to the lower collection compartment 500 comprises a descending ramp element accommodated in the migration pipe 400 configured to convey the migrating larvae.

These larvameter sensors comprise an array of nineteen leds facing an array of nineteen phototransistors at a distance of 47.5 mm from each other and mounted on an electronic card complete with the necessary components for enabling the sensor to interoperate with the firmware used to acquire the signals generated by it and to interoperate with the intelligent control unit.

The sensor 700 is characterized in that it is capable of detecting any obstructions of a light "blade" generated by the led array and received by its corresponding phototransistor array which is 47.5 mm spaced away.

In this version, the light "blade" comprises nineteen light beams which are spaced away by 1.25 mm from each other.

The scanning necessary to detect the passage of falling larvae through the light "blade" is obtained thanks to an array switch-on algorithm, characterized by switch-on commands sent to the individual leds and to the individual corresponding phototransistor, sequentially all along the led array.

A complete cycle covering the activation of the nienteen beams is repeated at a frequency of 3000 Hz.

Once a complete cycle is read, a nineteen-character long binary string is provided.

An uninterrupted reading results in a string of nineteen values ONE. Every beam obstruction results in a ZERO in its corresponding position.

The firmware analyses the string and measures the number of "objects" that crossed the beam.

Said device is provided with at least one temperature and humidity sensor 900 for assessing the environmental conditions present inside the composter.

In addition to the optoelectronic sensors and the temperature and humidity sensors, the device according to this invention also comprises a camera 800, which allows shape, colour, and magnitude recognition of said larvae present in the collection drawer 501; in the preferred embodiment of this invention, said camera is mounted in the collection drawer 501; this drawer can be removed and allow to transfer the larvae accumulated therein.

Upon migrating to try and find a site suitable for their metamorphosis, larvae also undergo initial morphological mutations, such as colour changes, and feature a given size; said camera is capable of identifying colour, size, and shape, to recognize the actual larval status, which might also be an index of alterations internally to the composter and/or alterations in the population of dipterans present in the composter and/or alterations in the environmental conditions.

Usually, when the density of the dipteran population is too high with respect to the quantity of waste that is fed, a competition takes place for nourishment, which might lead to two potentially harmful situations for a domestic composter: a spontaneous departure of part of the population to compensate for the shortage of resources, and a reduction in the ponderal increase of the larvae. Vice versa, if the density of the bioconversion agents were too low, there would be an increased ponderal growth of larvae, to the detriment of bioconversion quality.

Said sensors are in communication with a data acquisition and communication device in communication with the external enviroment, which represents the intelligent part of the device according to the present patent application and will be referred to below as intelligent control unit.

Said device is provided with a software which allows to count the number of larvae that terminated their own growth cycle inside the composter and "self-collected" in a technical compartment, present on the bottom thereof, and to send a request for new larvae to the operating centre which the composter is in communication with, so that the staff in charge of maintaining the composter can deliver a new lot of newborn larvae and, if necessary, collect those larvae which are at the end of their own growth process, in order to replace them and make it possible to continue the bioconversion process inside the composter itself.

The control unit 600 is provided with software means configured for processing the values coming from at least one optoelectronic sensor means 700 and counting the larvae of migrating saprophagous insects; said software means are also configured for processing the values coming from at least one camera 800 and recognizing the larval stage of said larvae of saprophagous insects.

Finally, the control unit 600 is provided with software means suitable for processing at least one organic waste bioconversion quality index on the basis of the values coming from said sensors 700, 800 and 900, and sending a request for new larvae to an operating centre on the basis of said at least one organic waste bioconversion quality index and/or an alarm, should the bioconversion quality be low.

The software present in the intelligent control unit constantly interrogates the larvameter sensor and/or the camera to determine, through an algorithm also based on the detection of the climatic conditions existing outside and inside the composter, how the device according to the present invention is operating and the quality of the bioconversion taking place internally thereto; in addition, this software makes it possible to monitor the frequency of the inocula performed by a user, the instants in time when inocula are inserted, and the number of migrated larvae, and make forecasts on the need for new inocula, if any.

Whenever a need for new inocula is detected by the control unit, this one sends a request to the operation centre accordingly.

The base of the composter also comprises a compartment 502 for collecting the percolate resulting from the bioconversion process, which is in communication with the bottom part of the bioconversion area, via an appropriate filtration grid.

This collection compartment might be conveniently formed of a removable can.

The present disclosure also includes a process for monitoring bioconversion quality, which is implemented via the device 1 and comprises the following steps:
- detecting a first value related to the transit of migrating larvae, by using said optoelectronic sensor means 700;
- processing a first parameter related to the number of larvae of migrating saprophagous insects by using said software means resident in said electronic processor means of the control unit 600.

This process also comprises the following steps:
- detecting at least one second value related to the shape, colour, and size of said migrating larvae, by using said sensor means 800;
- processing a parameter related to the recognition of the larva stage of the migrating larvae, by using said software means resident in said electronic processor means of the control unit 600.

Finally, the process comprises the following steps:
- detecting temperature and humidity values by using said sensor means 900;
- processing a third parameter related to the environmental conditions existing inside the device 1, by using said software means resident in said electronic processor means of the control unit 600.

Steps are also provided for determining a bioconversion quality index based on said at least one first, second, and/or third parameters as detected above and for sending a request for new larvae to an operating centre and/or an alarm, should said so determined bioconversion quality index be lower than a predetermined threshold value.

## Claims

1. An intelligent device (1) for composting organic waste by use of larvae of saprophagous insects, comprising an outer casing (100) which delimits an inner compartment (200) suitable for housing an inner container (300) coaxially placed inside the outer casing (100) and suitable for containing organic waste and larvae of saprophagous insects, **characterized in that** said inner container (300) is open on the top and is provided with a plurality of through holes (301) on the upper portion for making it possible for migrating larvae to pass from the inner container (300) to a migration pipe (400) up to reaching a lower collection compartment (500) placed between the bottom of the outer casing (100) and the bottom (302) of the inner container (300), suitable for collecting the percolate and the migrating larvae, **and in that** said device (1) is provided with optoelectronic sensor means (700) in the migration pipe (400) capable of detecting the passage of at least one migrating larva from the inner container (300), passing through the migration pipe (400) up to the lower collection compartment (500), said sensors being positioned along at least one migration route followed by the larvae from the inner container (300) to the lower collection compartment (500), said device being also provided with a control unit (600) comprising software means and a wireless module.

2. The intelligent device (1) according to claim 1, **characterized in that** said at least one migration route is provided with a descending ramp element accommodated in the migration pipe (400) configured for conveying the migrating larvae coming from said through holes (301) towards the lower collection compartment (500).

3. The intelligent device (1) according to claims 1 and 2, **characterized in that** said device is provided with air inlet/outlet means (101.1), wild dipteran egg laying means (103), and air conditioning means (201) connectable to at least one pipe (201.3) located in the inner container (300).

4. The intelligent device (1) according to any of the preceding claims, **characterized in that** said device features a substantially cylindrical shape and said migration pipe (400) is internally provided with at least one descending annular ramp element (401), tilted with respect to a horizontal plane, so that it has an opening in the point closest to the vertex as to allow the migrating larvae sliding and passing in a collection drawer (501) located in said lower collection compartment (500), and **in that** said optoelectronic sensor means (700) are located close to the ends (401.1 and 401.2) of said annular tilted ramp element (401).

5. The intelligent device (1) according to anyone of the preceding claims, **characterized in that** is provided with at least one temperature and humidity sensor mean (900) in the inner compartment (200).

6. The intelligent device (1) for composting organic waste according to anyone of the preceding claims **characterized in that** at least one camera (800) is provided in the lower collection compartment (500) and/o collection drawer (501), being capable to recognize shape, colour, and size of the migrating larvae.

7. The intelligent device (1) for composting organic waste according to anyone of the preceding claims, **characterized in that** said control unit (600) is provided with software means configured for processing the values coming from at least one optoelectronic sensor mean (700) and to perform counting of migrating saprophagous insects larvae.

8. The intelligent device (1) for composting organic waste according to anyone of the preceding claims, **characterized in that** said control unit (600) is provided with software means configured for processing the values coming from said at least one camera (800) and to perform the larval stage identification of said saprophagous insects larvae.

9. The intelligent device (1) for composting organic waste according anyone of the preceding claims, **characterized in that** said control unit (600) is provided with software means configured for processing at least one organic waste bioconversion quality index on the basis of the values coming from said sensor means (700; 800; 900), and sending a request for new larvae amount to an operation centre on the basis of said at least one organic waste bioconversion quality index and/or sending an alarm in case of low bioconversion quality.

10. A process for monitoring the quality of the bioconversion implemented by means of the device (1) according to claims 1 to 9, comprising the following steps:
- detecting a first value related to the passage of migrating larvae, by using said optoelectronic sensor means (700);
- processing a first parameter relating to the number of larvae of migrating saprophagous insects, by means of said software means residing in said electronic processor means of the control unit (600).

11. The process implemented by means of the device (1) according to claim 10, comprising the following steps:
- detecting at least one value related to the shape, colour, and size of said migrating larvae, by means of said camera (800);
- processing a second parameter related to the larval stage of the migrating larvae, by means of said software means resident in said electronic processor means of the control unit (600).

12. The process according to claims 10 and 11, **characterized in that** it comprises the following further steps:
- detecting temperature and humidity values by means of said sensor means (900);
- processing a third parameter related to the environmental conditions inside the device (1), by means of said software means resident in said electronic processor means of the control unit (600).

13. The process according to claims 9 to 12, **characterized in that** it comprises the steps of processing a bioconversion quality index on the basis of said at least one first, at least one second and/or at least one third parameter detected, and forwarding a request for new larvae to an operation centre and/or an alarm if said bioconversion quality index detected is lower than a predetermined threshold value.

## Patentansprüche

1. Intelligente Vorrichtung (1) zum Kompostieren von organischen Abfällen unter Verwendung von Larven saprophager Insekten, die ein Außengehäuse (100) umfasst, das einen Innenraum (200) abgrenzt, der zur Aufnahme eines Innenbehälters (300) geeignet ist, der koaxial innerhalb des Außengehäuses (100) angeordnet ist und zur Aufnahme von organischen Abfällen und Larven saprophager Insekten geeignet ist, **dadurch gekennzeichnet, dass** der Innenbehälter (300) oben offen ist und im oberen Bereich mit einer Vielzahl von Durchgangslöchern (301) versehen ist, um es wandernden Larven zu ermöglichen, vom Innenbehälter (300) zu einem Wanderrohr (400) zu gelangen, bis sie ein unteres Sammelfach (500) erreichen, das zwischen dem Boden des Außengehäuses (100) und dem Boden (302) des Innenbehälters (300) angeordnet ist und zum Auffangen des Perkolats und der wandernden Larven geeignet ist, und dass die Vorrichtung (1) mit opto-elektronischen Sensormitteln (700) im Wanderrohr (400) versehen ist, die den Durchgang von mindestens einer wandernden Larve vom Innenbehälter (300) durch das Wanderrohr (400) bis zum unteren Sammelfach (500) erfassen können, wobei die Sensoren entlang mindestens einer Wanderroute angeordnet sind, der die Larven vom Innenbehälter (300) zum unteren Sammelfach (500) folgen, wobei die Vorrichtung außerdem mit eine Steuereinheit (600), die Softwaremittel und ein Funkmodul umfasst, versehen ist.

2. Intelligente Vorrichtung (1) gemäß Anspruch 1, **dadurch gekennzeichnet, dass** die mindestens eine Wanderroute mit einem absteigenden Rampenelement versehen ist, das in dem Wanderrohr (400) untergebracht ist und dazu ausgelegt ist, die wandernden Larven, die aus den Durchgangslöchern (301) kommen, in Richtung des unteren Sammelfachs (500) zu befördern.

3. Intelligente Vorrichtung (1) gemäß den Ansprüchen 1 und 2, **dadurch gekennzeichnet, dass** die Vorrichtung mit Lufteinlass-/-auslassmitteln (101.1), mit Mitteln (103) zum Ablegen von Eiern wilder Zweiflügler und mit Klimaanlagen (201) versehen ist, die an mindestens ein Rohr (201.3) angeschlossen werden können, das sich im Innenbehälter (300) befindet.

4. Intelligente Vorrichtung (1) gemäß einem der vorhergehenden Ansprüche, **dadurch gekennzeichnet, dass** die Vorrichtung eine im Wesentlichen zylindrische Form aufweist und das Wanderrohr (400) im Inneren mit mindestens einem absteigenden ringförmigen Rampenelement (401) versehen ist, das in Bezug auf eine horizontale Ebene geneigt ist, so dass es an dem Punkt, der dem Scheitelpunkt am nächsten ist, eine Öffnung aufweist, um es den wandernden Larven zu ermöglichen, in eine Sammelschublade (501) zu gleiten und zu gelangen, die sich im unteren Sammelfach (500) befindet, und dadurch, dass die opto-elektronischen Sensormittel (700) in der Nähe der Enden (401.1 und 401.2) des ringförmigen geneigten Rampenelements (401) angeordnet sind.

5. Intelligente Vorrichtung (1) nach einem der vorhergehenden Ansprüche, **dadurch gekennzeichnet, dass** es mit mindestens einem Temperatur- und Feuchtigkeitssensor (900) im inneren Fach (200) ausgestattet ist.

6. Intelligente Vorrichtung (1) zur Kompostierung organischer Abfälle nach einem der vorhergehenden Ansprüche, **dadurch gekennzeichnet, dass** im unteren Sammelfach (500) und/oder in der Sammelschublade (501) mindestens eine Kamera (800) vorhanden und in der Lage ist, Form, Farbe und Größe wandernder Larven zu erkennen.

7. Intelligente Vorrichtung (1) zum Kompostieren von organischen Abfällen nach einem der vorhergehenden Ansprüche, **dadurch gekennzeichnet, dass** die Steuereinheit (600) mit Softwaremitteln ausgestattet ist, die so konfiguriert sind, dass sie die von mindestens einem opto-elektronischen Sensor (700) kommenden Werte verarbeiten können und um wandernde Larven von saprophagen Insekten zu zählen.

8. Intelligente Vorrichtung (1) zum Kompostieren von organischen Abfällen nach einem der vorhergehenden Ansprüche, **dadurch gekennzeichnet, dass** die Steuereinheit (600) mit Softwaremitteln ausgestattet ist, die so konfiguriert sind, dass sie die von der mindestens einen Kamera (800) kommenden Werte verarbeiten können und die Identifizierung der genannten saprophagen Insektenlarven im Larvenstadium durchzuführen.

9. Intelligente Vorrichtung (1) zur Kompostierung von organischen Abfällen nach einem der vorhergehenden Ansprüche, **dadurch gekennzeichnet, dass** die Steuereinheit (600) mit Softwaremitteln ausgestattet ist, die so konfiguriert sind, dass sie mindestens einen Biokonversionsqualitätsindex für organische Abfälle verarbeiten, basierend auf den Werten von dem Sensor (700, 800, 900) kommend, und um eine Anfrage für eine neue Menge an Larven an ein Operationszentrum zu übermitteln, basierend auf dem mindestens einen Biokonversionsqualitätsindex von organischen Abfällen und/oder um einen Alarm im Falle einer niedrigen Biokonversionsqualität zu senden.

10. Verfahren zur Überwachung der Qualität der mittels der Vorrichtung (1) gemäß den Ansprüchen 1 bis 9 erreichten Biokonversion, umfassend die folgenden Schritte:
- Erfassen eines ersten Werts in Bezug auf den Durchgang wandernder Larven unter Verwendung des opto-elektronischen Sensors (700);
- Verarbeiten eines ersten Parameters, der sich auf die Anzahl der Larven von saprophagen wandernden Insekten bezieht, mittels der im elektronischen Prozessor der Steuereinheit (600) vorhandenen Software.

11. Verfahren, mittels der Vorrichtung (1) nach Anspruch 10 durchgeführt und umfassend die folgenden Schritte:
- Erfassen von mindestens einem Wert in Bezug auf die Form, Farbe und Größe der wandernden Larven mittels der Kamera (800);
- Verarbeiten eines zweiten Parameters, der sich auf das Larvenstadium der wandernden Larven bezieht, mittels der in dem elektronischen Prozessor der Steuereinheit (600) befindlichen Software.

12. Verfahren nach den Ansprüchen 10 und 11, **dadurch gekennzeichnet, dass** es folgende weitere Schritte umfasst:
- Erfassen von Temperatur- und Feuchtigkeitswerten mittels des genannten Sensors (900);
- Verarbeiten eines dritten Parameters, der sich auf die Umgebungsbedingungen innerhalb der Vorrichtung (1) bezieht, mittels der im elektronischen Prozessor der Steuereinheit (600) befindlichen Software.

13. Verfahren nach den Ansprüchen 9 bis 12, **dadurch gekennzeichnet, dass** es die Schritte der Verarbeitung eines Biokonversionsqualitätsindex auf der Grundlage von mindestens einem ersten, mindestens einem zweiten und/oder mindestens einem dritten erfassten Parameter und der Übermittlung einer Anforderung neuer Larven an ein Operationszentrum und/oder eines Alarms, wenn der festgestellte Biokonversionsqualitätsindex unter einem vorab festgelegten Schwellenwert liegt.

## Revendications

1. Dispositif intelligent (1) de compostage de déchets organiques par utilisation de larves d'insectes saprophages, comprenant une enveloppe extérieure (100) qui délimite un compartiment intérieur (200) apte à loger un récipient (300) placé coaxialement à l'intérieur de l'enveloppe extérieure (100) et apte à contenir des déchets organiques et des larves d'insectes saprophages, **caractérisé en ce que** ledit récipient intérieur (300) est ouvert sur le dessus et est pourvu d'une pluralité de trous traversants (301) sur la partie supérieure pour permettre aux larves migratrices de passer du récipient intérieur (300) à un conduit de migration (400) jusqu'à atteindre un compartiment de collecte inférieur (500) placé entre le fond de l'enveloppe extérieure (100) et le fond (302) du récipient intérieur (300) apte à collecter le percolat et les larves migratrices, et **en ce que** ledit dispositif (1) est pourvu de moyens capteurs optoélectroniques (700) dans le conduit de migration (400) aptes à détecter le passage d'au moins une larve migratrice du récipient intérieur (300), en passant par le conduit de migration (400) jusqu'au compartiment de collecte inférieur (500), lesdits capteurs étant positionnés le long d'au moins une voie de migration suivie par les larves du récipient intérieur (300) au compartiment de collecte inférieur (500), ledit dispositif étant également pourvu d'une unité de commande (600) comprenant des moyens logiciels et un module sans fil.

2. Dispositif intelligent (1) selon la revendication 1, **caractérisé en ce que** ladite au moins une voie de migration est pourvue d'un élément de rampe descendante logé dans le conduit de migration (400), configuré pour transporter les larves migratrices provenant desdits trous traversants (301) vers le compartiment de collecte inférieur (500).

3. Dispositif intelligent (1) selon les revendications 1 et 2, **caractérisé en ce que** ledit dispositif est pourvu de moyens d'entrée/sortie d'air (101.1), de moyens de ponte d'oeufs de diptères sauvages (103) et de moyens de climatisation (201) pouvant être raccordés à au moins un conduit (201.3) disposé dans le conteneur interne (300).

4. Dispositif intelligent (1) selon l'une quelconque des revendications précédentes, **caractérisé en ce que** ledit dispositif présente une forme sensiblement cylindrique et ledit conduit de migration (400) est pourvu intérieurement d'au moins un élément de rampe annulaire descendant (401), incliné par rapport à un plan horizontal, de sorte qu'il présente une ouverture au point le plus proche du sommet de manière à permettre aux larves migratrices de glisser et de passer dans un bac de collecte (501) situé à l'intérieur du compartiment de collecte inférieur (500) et **en ce que** lesdits moyens de capteur optoélectronique (700) sont situés à proximité des extrémités (401.1 et 401.2) de l'élément de rampe annulaire incliné (401).

5. Dispositif intelligent (1) selon l'une quelconque des revendications précédentes, **caractérisé en ce qu'**il est pourvu d'au moins un capteur de température et d'humidité (900) dans le compartiment interne (200).

6. Dispositif intelligent (1) de compostage de déchets organiques selon l'une quelconque des revendications précédentes, **caractérisé en ce qu'**au moins une caméra (800) est obtenue dans le compartiment de collecte inférieur (500) et/ou dans le tiroir de collecte (501), et est capable de reconnaître la forme, la couleur et la taille des larves migratrices.

7. Dispositif intelligent (1) de compostage de déchets organiques selon l'une quelconque des revendications précédentes, **caractérisé en ce que** ladite unité de commande (600) est pourvue de moyens logiciels configurés pour traiter les valeurs provenant d'au moins un capteur optoélectronique (700) et de compter les larves migratrices d'insectes saprophages.

8. Dispositif intelligent (1) de compostage de déchets organiques selon l'une quelconque des revendications précédentes, **caractérisé en ce que** ladite unité de commande (600) est pourvue de moyens logiciels configurés pour traiter les valeurs provenant de ladite au moins une caméra (800) et de procéder à l'identification au stade larvaire desdites larves d'insectes saprophages.

9. Dispositif intelligent (1) de compostage de déchets organiques selon l'une quelconque des revendications précédentes, **caractérisé en ce que** ladite unité de commande (600) est pourvue de moyens logiciels configurés pour traiter au moins un indice de qualité de bioconversion des déchets organiques, sur la base des valeurs provenant dudit capteur (700, 800, 900) et de transmettre une demande de nouvelle quantité de larves à un centre opérationnel en fonction dudit au moins un indice de qualité de bioconversion des déchets organiques et/ou de transmettre une alarme en cas de faible qualité de la bioconversion.

10. Procédé de contrôle de la qualité de bioconversion réalisée au moyen du dispositif (1) selon les revendications 1 à 9, comprenant les opérations suivantes:
- détecter une première valeur relative au passage de larves migratrices, à l'aide dudit capteur optoélectronique (700);
- traiter un premier paramètre relatif au nombre de larves d'insectes migrateurs saprophages, au moyen dudit logiciel résidant dans ledit processeur électronique de l'unité de commande (600).

11. Procédé réalisé au moyen du dispositif (1) selon la revendication 10, comprenant les opérations suivantes:
- détecter au moins une valeur relative à la forme, à la couleur et à la taille desdites larves migratrices, au moyen de ladite caméra (800);
- traiter un deuxième paramètre relatif au stade larvaire des larves migratrices, au moyen dudit logiciel résidant dans ledit processeur électronique de l'unité de commande (600).

12. Procédé selon les revendications 10 et 11, **caractérisé en ce qu'**il comprend les opérations supplémentaires suivantes:
- détecter des valeurs de température et d'humidité au moyen dudit capteur (900);
- traiter un troisième paramètre relatif aux conditions environnementales à l'intérieur du dispositif (1) au moyen dudit logiciel résidant dans ledit processeur électronique de l'unité de commande (600).

13. Procédé selon les revendications 9 à 12, **caractérisé en ce qu'**il comprend les opérations d'élaboration d'un indice de qualité de bioconversion à partir d'au moins un premier, au moins un deuxième et/ou au moins un troisième paramètre détecté, et de transmission d'une demande de nouvelles larves à un centre opérationnel et/ou d'alarme si l'indice de qualité de bioconversion détecté est inférieur à une valeur seuil préétablie.
